Europäisches Patentamt

**European Patent Office**

Office européen des brevets

(11) Numéro de publication: **0 290 299 B1**

## FASCICULE DE BREVET EUROPEEN

(45) Date de publication de fascicule du brevet: **10.03.93**

(51) Int. Cl.⁵: **A61K 9/16**, A61K 35/78, A61K 47/44

(21) Numéro de dépôt: **88400760.0**

(22) Date de dépôt: **29.03.88**

(54) **Forme galénique à base d'extrait sec de plantes.**

(30) Priorité: **03.04.87 FR 8704700**

(43) Date de publication de la demande:
**09.11.88 Bulletin 88/45**

(45) Mention de la délivrance du brevet:
**10.03.93 Bulletin 93/10**

(84) Etats contractants désignés:
**AT BE CH DE ES GB GR IT LI LU NL SE**

(56) Documents cités:
**FR-A- 840 092**
**FR-A- 2 007 352**
**FR-A- 2 145 803**
**FR-A- 2 583 640**
**US-A- 4 344 934**

**S.T.P. PHARMA, vol. 1, no. 6, 1985;**
**C.CHEMTOB, pp. 531-538**

(73) Titulaire: **PIERRE FABRE MEDICAMENT**
**45, Place Abel Gance**
**F-92100 Boulogne(FR)**

(72) Inventeur: **Dupinay, Pierre**
**4, rue de la République**
**F-81200 Mazamet(FR)**

(74) Mandataire: **Ahner, Francis et al**
**CABINET REGIMBEAU, 26, avenue Kléber**
**F-75116 Paris (FR)**

**EP 0 290 299 B1**

## Description

La présente invention concerne un procédé de préparation d'une forme galénique solide se présentant sous la forme de grains hydrosolubles contenant un principe actif qui, à l'état brut, est insoluble dans l'eau.

Plus particulièrement, la présente invention a eu pour but de préparer, à partir d'un extrait sec de Ginkgo biloba, une forme pharmaceutique ambulatoire, se présentant sous la forme d'une prise unitaire destinée à l'administration par voie orale, sous forme d'une poudre dispersable dans l'eau froide. Bien entendu, une telle forme galénique doit impérativement respecter l'intégrité du principe actif, en particulier l'extrait sec de Ginkgo biloba, et rester stable dans le temps.

Normalement, un principe actif tel qu'un extrait sec de plantes, en particulier un extrait sec de Ginkgo biloba, se réagglomère dans l'eau pour former des grumeaux noirâtres impossible à disperser.

En outre, le simple mélange d'un extrait sec de Ginkgo biloba avec un diluant conduit à une hétérogénéité du principe actif dans la masse, en raison d'une différence de densité et de granulométrie des deux poudres. Au surplus, un tel mélange, lorsqu'il est mis au contact de l'eau, se sépare avec relargage de l'extrait de Ginkgo biloba et formation de grumeaux.

La présente invention a donc eu pour but de rendre un tel extrait végétal parfaitement dispersable dans l'eau et aisément manipulable en vue de sa mise sous forme de dosage unitaire. Dans le cas d'un extrait sec de Ginkgo biloba, le principe actif est utilisé par exemple à une dose unitaire de 40 mg.

Conformément à la présente invention, le procédé de préparation d'une forme galénique solide contenant un principe actif insoluble dans l'eau, se présentant sous la forme de grains hydrosolubles, est caractérisé en ce que l'on réalise les opérations successives suivantes :
- humectation et/ou solubilisation du principe actif au moyen d'un solvant alcoolique au hydroalcoolique ;
- addition, au principe actif humecté ou solubilisé, d'un agent tensioactif et éventuellement d'un liant ;
- homogénéisation de la suspension ou solution ainsi obtenue ou solubilisation ;
- mélange de ladite suspension ou solution avec un support hydrosoluble pharmaceutiquement acceptable et compatible avec le principe actif;
- granulation dudit mélange et évaporation du solvant par séchage.

Le procédé selon l'invention vise la préparation d'une forme galénique à base d'un extrait sec de plantes. A titre d'autres exemples de principes actifs susceptibles d'entrer dans les formes galéniques solides selon l'invention, on mentionnera par exemple la dihydroergotamine ainsi que la raubasine.

Selon une autre caractéristique de la présente invention, l'humectation du principe actif est réalisée à l'aide d'un mélange eau-alcool, en particulier à l'aide d'un mélange eau-éthanol.

Dans le cas où l'on ne réalise pas une simple humectation du principe actif mais que l'on effectue une solubilisation complète de ce principe actif, on utilisera de préférence de l'éthanol ou encore un mélange eau-éthanol. Le mouillage et/ou la solubilisation du principe actif sera avantageusement obtenu(s) en utilisant 0,250 à 25 litres de solvant par kg de principe actif traité.

Selon une autre caractéristique du procédé, l'agent tensioactif utilisé est de préférence un agent du type anionique ou non-ionique, en particulier choisi parmi le laurylsulfate de sodium, les polysorbates ainsi que les huiles de ricin hydrogénées polyoxyéthylénées. De préférence, le rapport pondéral entre l'agent tensioactif et le principe actif est compris entre 0,01 et 5.

Le liant facultativement utilisé dans le cadre du présent procédé, est un liant hydrosoluble de préférence choisi parmi la polyvidone, l'hydroxypropylcellulose, l'hydroxypropylméthylcellulose, le saccharose, le polyéthylèneglycol ou encore la gélatine. De préférence, on utilise jusqu'à environ 10% en poids de liant par rapport au poids total des constituants en mélange.

Selon une autre caractéristique essentielle du procédé selon l'invention, on fixe le principe actif sur un support hydrosoluble pharmaceutiquement acceptable et compatible avec le principe actif, de préférence choisi parmi le saccharose, le lactose, le mannitol et les malto-dextrines. Avantageusement, le rapport pondéral entre le support et le principe actif est compris entre 2,5 et 75.

L'operation de granulation sera avantageusement conduite dans un mélangeur-granulateur, ou encore par pulvérisation dans un lit fluidisé. L'addition du liant hydrosoluble précédemment cité, permet dans la pratique d'améliorer la granulation du mélange.

En variante, les opérations de granulation et de séchage pourront être mises en oeuvre simultanément au cours d'une opération unique de lyophilisation. Dans pareil cas, l'étape de solubilisation sera de préférence réalisée à l'aide d'un alcool tel que l'éthanol.

L'étape ultime de séchage des grains peut être obtenue par évaporation à des températures de l'ordre de 35 à 50°C, le cas échéant, sous un vide partiel, en fonction de la nature du solvant utilisé. Après séchage, on obtient, conformément au procédé de l'invention, une poudre de coloration homogène au sein

2

de laquelle l'extrait sec de Ginkgo biloba se trouve fixé sur son support, ce qui permet d'éliminer ainsi tous risques de démixtion. Avantageusement, après l'opération de séchage des grains, on procédé à une opération de calibrage qui est avantageusement conduit sur une grille présentant une ouverture de mailles d'environ 0,8 mm. On obtient ainsi des grains qui s'écoulent librement, ce qui permet en outre une répartition très régulière du poids du principe actif à doser dans les sachets.

La présente invention concerne également, à titre de produits industriels, les formes galéniques obtenues par la mise en oeuvre du procédé décrit ci-dessus.

La forme galénique ainsi obtenue, présente non seulement une bonne aptitude à la dispersion dans l'eau froide, mais également une dissolution totale et instantanée. Cette solubilisation reste stable dans le temps ; on ne constate ni relargage, ni précipitation de l'extrait sec.

On obtient donc par dissolution dans l'eau, une solution d'extrait de Ginkgo biloba, ce qui est particulièrement intéressant sur le plan pharmaceutique, car il y a bioéquivalence de la forme solide avec une solution vraie de Ginkgo biloba. En outre, la stabilité physico-chimique et microbiologique d'une poudre est bien sûr nettement supérieure à celle d'une solution.

A titre d'exemples de telles formes galéniques solides se présentant sous forme de grains hydrosolubles, on mentionnera ci-dessous une forme de dosage unitaire comprenant :

| | |
|---|---|
| extrait sec de Ginkgo biloba environ | 40 mg |
| support | 100 à 3000 mg |
| tensioactif | 0,4 à 200 mg |
| liant | 0 à 200 mg |
| édulcorant | q.s. |
| arôme soluble | q.s. |

L'invention concerne plus particulièrement la forme de dosage unitaire suivante :

| | |
|---|---|
| extrait sec de Ginkgo biloba | 40 mg |
| mannitol | 2000 mg |
| huile de ricin hydrogénée polyoxyéthylénée | 100 mg |
| saccharose | 105 mg |
| édulcorant | q.s. |
| arôme soluble | q.s. |

La granulométrie de cet exemple particulier de forme galénique selon l'invention est indiquée ci-dessous :

## Granulométrie du produit fini

|  |  |  |  |  |
|---|---|---|---|---|
| 1 mm | < 0% | | | |
| 0,630 mm | < environ | 30% | < | 1 mm |
| 0,400 mm | < environ | 20% | < | 0,630 mm |
| 0,250 mm | < environ | 15% | < | 0,400 mm |
| 0,100 mm | < environ | 25% | < | 0,250 mm |
| 0,050 mm | < environ | 10% | < | 0,100 mm |
| | 0% | < | 0,050 mm | |

Sur cette forme galénique particulière de Ginkgo biloba, des expérimentations de solubilisation comparées ont été effectuées. Les résultats ont été consignés dans le tableau ci-après, qui révèlent l'amélioration décisive apportée par la présente invention.

|  | Solubilité dans l'eau (50 ml à 20°C) |
|---|---|
| Extrait sec de Ginkgo biloba | NULLE<br>Formation de grumeaux d'extrait |
| Extrait sec de Ginkgo biloba + support (simple mélange physique) | NULLE<br>Démixtion<br>Formation de grumeaux d'extrait |
| Extrait sec de Ginkgo biloba fixé sur support (selon la présente invention) | TOTALE<br>SOLUTION LIMPIDE |

## Revendications

1. Procédé de préparation d'une forme galénique solide contenant un principe actif insoluble dans l'eau, se présentant sous la forme de grains hydrosolubles, caractérisé en ce que ledit principe actif insoluble dans l'eau est un extrait sec de plantes, en particulier un extrait de Ginkgo biloba, et en ce que l'on réalise les opérations successives suivantes :
   - humectation et/ou solubilisation du principe actif au moyen d'un solvant alcoolique ou hydroalcoolique ;
   - addition, au principe actif humecté ou solubilisé, d'un agent tensioactif et éventuellement d'un liant ;
   - homogénéisation de la suspension ou solution ainsi obtenue ou solubilisation ;
   - mélange de ladite suspension ou solution avec un support hydrosoluble pharmaceutiquement acceptable et compatible avec le principe actif ;
   - granulation dudit mélange et évaporation du solvant par séchage.

2. Procédé selon la revendication 1, caractérisé en ce que l'humectation du principe actif est réalisée à l'aide d'un mélange eau-alcool, en particulier à l'aide d'un mélange eau-éthanol.

3. Procédé selon la revendication 1, caractérisé en ce que la solubilisation du principe actif est réalisée au moyen d'éthanol ou bien d'un mélange eau-éthanol.

4. Procédé selon la revendication 3, caractérisé en ce que l'agent tensioactif est un agent non-ionique ou anionique, en particulier choisi parmi le laurylsulfate de sodium, les polysorbates et les huiles de ricin hydrogénées polyoxyéthylénées.

5. Procédé selon l'une des revendications 1 à 4, caractérisé en ce que le liant est hydrosoluble, et de préférence choisi parmi la polyvidone, l'hydroxypropylcellulose, l'hydroxypropylméthylcellulose, le saccharose, le polyéthylèneglycol et la gélatine.

6. Procédé selon l'une des revendications 1 à 5, caractérisé en ce que le support hydrosoluble est choisi parmi le saccharose, le lactose, le mannitol et les malto-dextrines.

7. Procédé selon l'une des revendications 1 à 6, caractérisé en ce que la granulation est réalisée dans un mélangeur-granulateur ou par pulvérisation dans un lit fluidisé.

8. Procédé selon l'une des revendications 1 à 6, caractérisé en ce que les opérations de granulation et de séchage sont mises en oeuvre simultanément au cours d'une opération de lyophilisation.

**9.** Procédé selon l'une des revendications 1 à 8, caractérisé en ce que, après le séchage des grains, on procède à une opération de calibrage, de préférence sur une grille présentant une ouverture de maille d'environ 0,8 mm.

**10.** Procédé selon l'une des revendications 1 à 9, caractérisé en ce que l'on mouille et/ou solubilise le principe actif en uitilisant ledit solvant à raison de 0,250 à 2,5 litres de solvant par kg de principe actif.

**11.** Procédé selon l'une des revendications 1 à 10, caractérisé en ce que le rapport pondéral entre le support et le principe actif est compris entre 2,5 et 75.

**12.** Procédé selon l'une des revendications 1 à 11, caractérisé en ce que le rapport pondéral entre l'agent tensioactif et le principe actif est compris entre 0,01 et 5.

**13.** Procédé selon l'une des revendications 1 à 12, caractérisé en ce que l'on utilise jusqu'à environ 10 % en poids de liant par rapport au poids total des constituants en mélange.

**14.** Forme galénique solide, sous forme de grains hydrosolubles, caractérisée en ce qu'elle se présente sous une forme de dosage unitaire comprenant :

| | |
|---|---|
| extrait sec de Ginkgo biloba environ | 40 mg |
| support | 100 à 3000 mg |
| tensioactif | 0,4 à 200 mg |
| liant | 0 à 200 mg |
| édulcorant | q.s. |
| arôme soluble | q.s. |

**15.** Forme galénique selon la revendication 14, caractérisée en ce qu'elle se présente sous la forme de dosage unitaire comprenant :

| | |
|---|---|
| extrait sec de Ginkgo biloba | 40 mg |
| mannitol | 2000 mg |
| huile de ricin hydrogénée polyoxyéthylénée | 100 mg |
| saccharose | 105 mg |
| édulcorant | q.s. |
| arôme soluble | q.s. |

**Claims**

**1.** A method for preparing a solid galenic form containing an active principle which is insoluble in water, in the form of water-soluble grains, characterised in that the said water-insoluble active principle is a dry plant extract, in particular an extract from Ginkgo biloba, and in that the following successive operations are performed:
- moistening and/or solubilisation of the active principle by means of an alcoholic or aqueous/alcoholic solvent,
- adding a surfactant with or without a binder to the moistened or solubilised active principle,
- homogenising the suspension or solution so obtained or solubilisation,
- mixing the said suspension or solution with a pharmaceutically acceptable water-soluble substrate which is compatible with the active principle,
- granulating the said mixture and evaporating the solvent by drying.

**2.** A method according to claim 1, characterised in that the active principle is moistened using a mixture of water and alcohol, in particular using a mixture of water and ethanol.

5

3. A method according to claim 1, characterised in that solubilisation of the active principle is achieved using ethanol or a mixture of ethanol and water.

4. A method according to claim 3, characterised in that the surfactant is a non-ionic or anionic surfactant, in particular selected from sodium lauryl sulphate, polysorbates and polyoxyethylenated hydrogenated castor oils.

5. A method according to one of claims 1 to 4, characterised in that the binder is water soluble, and is preferably selected from polyvidone, hydroxypropyl cellulose, hydroxypropyl methyl cellulose, saccharose, polyethylene glycol and gelatin.

6. A method according to one of claims 1 to 5, characterised in that the water-soluble substrate is selected from saccharose, lactose, mannitol and the malto-dextrins.

7. A method according to one of claims 1 to 6, characterised in that granulation is effected in a mixer-granulator or by powder formation in a fluidised bed.

8. A method according to one of claims 1 to 6, characterised in that the granulation and drying operations are carried out simultaneously in the course of a freeze-drying operation.

9. A method according to one of claims 1 to 8, characterised in that once the grains have been dried they are gauged preferably on a grid with a mesh of dimensions approximately 0.8 mm.

10. A method according to one of claims 1 to 9, characterised in that the active principle is wetted and/or solubilised using the said solvent in a ratio of 0.250 to 2.5 litres per kg of active principle.

11. A method according to one of claims 1 to 10, characterised in that the ratio between the substrate and the active principle lies between 2.5 and 75 by weight.

12. A method according to one of claims 1 to 11, characterised in that the ratio between the surfactant and the active principle lies between 0.01 and 5 by weight.

13. A method according to one of claims 1 to 12, characterised in that up to about 10% by weight of binder with respect to the total weight of the components of the mixture is used.

14. A solid galenic preparation in the form of water-soluble grains, characterised in that it takes the form of individual doses containing:

| | |
|---|---|
| Dry extract of Ginkgo biloba, approximately | 40 mg |
| Substrate | 100 to 3000 mg |
| Surfactant | 0.4 to 200 mg |
| Binder | 0 to 200 mg |
| Sweetener | q.s. |
| Soluble flavour | q.s. |

15. A galenic preparation according to claim 14, characterised in that it takes the form of individual doses containing:

| | |
|---|---|
| Dry extract of Ginkgo biloba | 40 mg |
| Mannitol | 2000 mg |
| Polyoxyethylenated hydrogenated castor oil | 100 mg |
| Saccharose | 105 mg |
| Sweetener | q.s. |
| Soluble flavour | q.s. |

**Patentansprüche**

1. Verfahren zur Herstellung eines festen galenischen Präparats in Form von wasserlöslichen Körnchen, das einen in Wasser unlöslichen Wirkstoff (aktives Prinzip) enthält, dadurch gekennzeichnet, daß der in Wasser unlösliche Wirkstoff ein Trockenextrakt von Pflanzen, insbesondere ein Extrakt von Ginko biloba, ist und daß man nacheinander die folgenden Operationen durchführt:
   - Anfeuchten (Benetzen) und/oder Solubilisieren des Wirkstoffes mit einem alkoholischen oder wäßrig-alkoholischen Lösungsmittel;
   - Zugabe eines Tensids und gegebenenfalls eines Bindemittels zu dem angefeuchteten (benetzten) oder solubilisierten Wirkstoff;
   - Homogenisieren oder Solubilisieren der so erhaltenen Suspension oder Lösung;
   - Mischen dieser Suspension oder Lösung mit einem pharmazeutisch akzeptablen und mit dem Wirkstoff verträglichen wasserlöslichen Träger; und
   - Granulieren der Mischung und Verdampfen des Lösungsmittels durch Trocknen.

2. Verfahren nach Anspruch 1, dadurch gekennzeichnet, daß das Anfeuchten (Benetzen) des Wirkstoffes mit einem Wasser/Alkohol-Gemisch, insbesondere mit einem Wasser/Ethanol-Gemisch, durchgeführt wird.

3. Verfahren nach Anspruch 1, dadurch gekennzeichnet, daß das Solubilisieren des Wirkstoffes mit Ethanol oder auch mit einem Wasser/Ethanol-Gemisch durchgeführt wird.

4. Verfahren nach Anspruch 3, dadurch gekennzeichnet, daß das Tensid ein nicht-ionisches oder anionisches Tensid ist, das insbesondere ausgewählt wird aus Natriumlaurylsulfat, Polysorbaten und hydrierten Polyoxyethylen-Rizinusölen.

5. Verfahren nach einem der Ansprüche 1 bis 4, dadurch gekennzeichnet, daß das Bindemittel wasserlöslich ist und daß es vorzugsweise ausgewählt wird aus der Gruppe Polyvidon, Hydroxypropylcellulose, Hydroxypropylmethylcellulose, Saccharose, Polyethylenglycol und Gelatine.

6. Verfahren nach einem der Ansprüche 1 bis 5, dadurch gekennzeichnet, daß der wasserlösliche Träger ausgewählt wird aus der Gruppe Saccharose, Lactose, Mannit und der Maltodextrinen.

7. Verfahren nach einem der Ansprüche 1 bis 6, dadurch gekennzeichnet, daß die Granulierung in einem Mischer-Granulator oder durch Pulverisierung in einer Wirbelschicht durchgeführt wird.

8. Verfahren nach einem der Ansprüche 1 bis 6, dadurch gekennzeichnet, daß die Granulierung und Trocknung gleichzeitig während einer Lyophilisierung durchgeführt werden.

9. Verfahren nach einem der Ansprüche 1 bis 8, dadurch gekennzeichnet, daß die Körnchen nach dem Trocknen klassiert werden, vorzugsweise auf einem Sieb, das eine Maschenweite von etwa 0,8 mm aufweist.

10. Verfahren nach einem der Ansprüche 1 bis 9, dadurch gekennzeichnet, daß man den Wirkstoff benetzt und/oder solubilisiert, durch Verwendung des Lösungsmittels in einer Menge von 0,250 bis 2,5 l Lösungsmittel pro kg Wirkstoff.

11. Verfahren nach einem der Ansprüche 1 bis 10, dadurch gekennzeichnet, daß das Gewichtsverhältnis zwischen dem Träger und dem Wirkstoff zwischen 2,5 und 75 liegt.

12. Verfahren nach einem der Ansprüche 1 bis 11, dadurch gekennzeichnet, daß das Gewichtsverhältnis zwischen dem Tensid und dem Wirkstoff zwischen 0,01 und 5 liegt.

13. Verfahren nach einem der Ansprüche 1 bis 12, dadurch gekennzeichnet, daß man bis zu etwa 10 Gew.-% Bindemittel, bezogen auf das Gesamtgewicht der Bestandteile der Mischung, verwendet.

14. Festes galenisches Präparat in Form von wasserlöslichen Körnchen, dadurch gekennzeichnet, daß es in einer Einheitsdosierungsform vorliegt, die enthält:

| Trockenextrakt von Ginko biloba | ca 40 mg |
|---|---|
| Träger | 100 bis 3000 mg |
| Tensid | 0,4 bis 200 mg |
| Bindemittel | 0 bis 200 mg |
| Süßungsmittel | q.s. |
| lösliches Aroma | q.s. |

**15.** Galenisches Präparat nach Anspruch 14, dadurch gekennzeichnet, daß es in einer Einheitsdosierungsform vorliegt, die enthält

| Trockenextrakt von Ginko biloba | 40 mg |
|---|---|
| Mannit | 2000 mg |
| hydriertes Polyoxyethylen-Rizinusöl | 100 mg |
| Saccharose | 105 mg |
| Süßungsmittel | q.s. |
| lösliches Aroma | q.s. |